(19) Europäisches Patentamt
European Patent Office
Office européen des brevets

(11) Veröffentlichungsnummer: **0 095 702 B1**

(12) # EUROPÄISCHE PATENTSCHRIFT

(45) Veröffentlichungstag der Patentschrift: **12.04.89**

(51) Int. Cl.⁴: **C 12 N 15/00**, C 12 P 21/02, C 07 H 21/04

(21) Anmeldenummer: **83105116.4**

(22) Anmeldetag: **24.05.83**

(54) **Mikrobiologisch hergestelltes Interferon alpha2(Arg), DNA-Sequenz, die für dieses Interferon codiert, Mikroorganismen, die diese genetische Information enthalten, und Verfahren zu ihrer Herstellung.**

(30) Priorität: **28.05.82 DE 3220116**

(43) Veröffentlichungstag der Anmeldung: **07.12.83 Patentblatt 83/49**

(45) Bekanntmachung des Hinweises auf die Patenterteilung: **12.04.89 Patentblatt 89/15**

(84) Benannte Vertragsstaaten: **AT BE CH DE FR GB IT LI LU NL SE**

(56) Entgegenhaltungen:
**EP-A- 0 028 033**
**EP-A- 0 034 306**
**EP-A- 0 034 307**
**EP-A- 0 051 873**
**EP-A- 0 076 489**
**DE-A- 2 906 160**
**DE-A- 3 043 981**
**DE-A- 3 226 319**
**GB-A- 2 068 970**
**GB-A- 2 071 108**

**Eur. J. Cell Biology (1981) Bd. 25, Seiten 8-9**
**Nature (1980) Bd. 287, S. 408-411**

(73) Patentinhaber: **Dr. Karl Thomae GmbH, Postfach 1755, D-7950 Biberach (Riss) (DE)**

(72) Erfinder: **Dworkin-Rastl, Eva, Dr., 90 Morning Side Drive Abt. 6/j., N.Y. New York 10027 (US)**
Erfinder: **Dworkin, Marc-Bruce, Dr., 90 Morning Side Drive Abt. 6/j., N.Y. New York 10027 (US)**
Erfinder: **Adolf, Günther, Dr. Mag., Johannagasse 20/7, A-1120 Wien (AT)**
Erfinder: **Meindl, Peter, Dr., Hockegasse 63/1, A-1120 Wien (AT)**
Erfinder: **Bodo, Gerhard, Prof. Dr., Belghofergasse 27, A-1120 Wien (AT)**
Erfinder: **Swetly, Peter, Dr., Hietzinger Hauptstrasse 40 B/9, A-1130 Wien (AT)**

(56) Entgegenhaltungen: (Fortsetzung)
**NATURE, Vol. 287, 2.Oktober 1980, USA D.GOEDDEL et a.: "Human leucocyte interferon produced by E.coli is biologically active" Seiten 411-415**
**NATURE, Vol. 284, No. 5754, 27.März 1980, USA S.NAGATA et al.:"Synthesis in E.coli of a polypeptide with human leucocyte activity" Seiten 316-320**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 095 702 B1

ACTORUM AG

## Beschreibung

Aus der Literatur sind drei Typen von Humaninterferonen bekannt, nämlich Leukocyten-Interferon (Interferon-α, Kurzbezeichnung: IFN-α), Fibroblasten-Interferon (Interferon-β, Kurzbezeichnung: IFN-β) und Immuninterferon (Interferon-γ, Kurzbezeichnung: IFN-γ) (siehe W.E. Stewart II «The Interferon System», Springer-Verlang Wien-New York, 2. Auflage (1981)). Menschliche Leukocyten oder menschliche myeloblastoide Zellen, welche mit Virus stimuliert sind, produzieren Leukocyten-Interferon, menschliche Fibroblasten, welche mit Virus oder einer geeigneten Nukleinsäure induziert sind, Fibroblasten-Interferon und menschliche T-Lymphocyten, welche mit Mitogen, z.B. Concanavalin, induziert sind, Immuninterferon.

Außerdem ist bekannt, daß menschliche Zellen des B-Lymphocyten-Typs, wie sie beispielsweise durch die Zellinien NC-37, Namalwa, Akuba oder RPMI 1788 repräsentiert werden, nach Stimulation durch Virus gleichzeitig Leukocyten-Interferon und Fibroblasten-Interferon produzieren (siehe Journal of General Virology 38, 51–59 (1977)). Hierbei können die Mengenverhältnisse an produziertem IFN-α und IFN-β durch die Wahl der Induktionsbedingungen variiert werden (siehe Journal of Interferon Research 2, 575–585 (1982)). Beispielsweise erhält man aus mit Sendaivirus induzierten Zellen verschiedener Zellinien folgende Mengenverhältnisse an IFN-α und IFN-β:

| Zellinie | Prozent Interferon-Aktivität, neutralisiert durch spezifische Antiseren gegen | | |
|---|---|---|---|
| | IFN-α | IFN-β | IFN-α + IFN-β |
| Namalwa | 52 | 34 | ⩾98 |
| NC-37 | 40 | 52 | ⩾97 |
| Akuba | 85 | 27 | ⩾98 |
| RPMI 1788 | 68 | 29 | ⩾98 |

Des weiteren brachte molekulares Klonieren von IFN-α-Genen aus Leukocyten (siehe Nature 284, 316–320 (1980); Science 209, 1343–1347 (1980) und EP-A1-0 032 134) und aus myeloblastoiden Zellen (siehe Nature 287, 411–416 (1980), ibid 290, 20–26 (1981) und GB-A-2 079 291) das Ergebnis, daß IFN-α durch eine Genfamilie codiert wird, welche aus mindestens 10 voneinander unterscheidbaren Genen besteht, was wiederum zur Folge hat, daß die Genprodukte dieser DNA-Sequenzen kein einheitliches Protein darstellen; das heißt, daß IFN-α eine Mischung aus einander ähnlichen Proteinen darstellt. Diese Subtypen wurden als IFN-α 1, 2, 3 .... (siehe Nature 287, 401–408 (1980) und Gene 15, 379–394 (1981)) oder LeIFN A, B, C..... (siehe Nature 290, 20–26 (1981) in der Literatur bezeichnet.

Demgegenüber wurde für IFN-β eine einheitliche DNA-Sequenz gefunden; das heißt, für Fibroblasteninterferon codiert nur ein Einzelgen, und es sind daher auch keine Subtypen bekannt (siehe Nature 285, 542–547 (1980)).

Obwohl, anders als innerhalb der IFN-α-Genfamilie, zwischen IFN-α-Genen und dem IFN-β-Gen keine Kreuzhybridisierung stattfindet, weisen die Sequenzen etwa 45% Homologie auf (siehe Nature 285, 547–549 (1980)). Hierbei ist der längste Sequenzabschnitt, bei welchem vollständige Homologie zwischen den (5 von 7) funktionellen IFN-α-Genen und dem IFN-β-Gen besteht, 13 Nukleotide lang.

Dieses Tridekanukleotid der Formel

5'–dCCTTCTGGAACTG–3'

wird in European Journal of Cell Biology 25, 8–9 (1981) beschrieben, wobei gleichzeitig vorgeschlagen wird, dieses synthetische Tridekanukleotid oder eine cDNA, die mit diesem Tridekanukleotid als Primer synthetisiert worden ist, als Hybridisierungsprobe zu verwenden, um eine Namalwa-Zellen cDNA-Klonbank durchzuforsten und die Klone, die Interferon-Sequenzen enthalten, zu identifizieren.

Die dieses Tridekanukleotid enthaltenden IFN-α-Gene sind LeIFN B, C, D, F und G; in LeIFN A und H sind jedoch nur 12 der 13 Nukleotide vorhanden (siehe Nature 290, 20–26 (1981)).

In der GB-A-2 068 970 wird analog vorgeschlagen, ausgewählte Oligonukleotide, z.B. ein Oligonukleotid, dessen Sequenz dem Fibroblasten- und Leukozyten-Interferon-Gen gemeinsam ist, zu verwenden, um verschiedene Interferon-Gene, die in menschlichen Chromosomen-Gen-Bänken enthalten sind oder in einem Restriktionsaufschluß von totaler genomischer DNA vorhanden sind, zu erfassen und zu isolieren.

Desweiteren wird in der EP-A-0 076 489 vorgeschlagen, ein radioaktiv markiertes, synthetisches Oligonukleotid, z.B. das obige Tridekanukleotid oder eine radioaktiv markierte α- und β-spezifische IFN-cDNA, z.B. eine cDNA, die mit dem genannten Tridekanukleotid als Primer synthetisiert worden ist, als Sonde zur Identifizierung von Kolonien, die chromosomale IFN-α- und IFN-β-Gene enthalten, zu verwenden.

Mit Hilfe des vorstehend erwähnten Tridekanukleotids konnte unter Verwendung der vorstehend erwähnten Methode der neue Klon IF7, der für das neue IFN-α2(Arg) codiert, aufgefunden werden. Dieser Klon enthält die für IFN-α2(Arg) codierende Teilsequenz der Formel

```
                120
                  C CTG GCA CAG ATG AGG AGA ATC TCT CTT TTC TCC   153
TGC TTG AAG GAC AGA CGT GAC TTT GGA TTT CCC CAG GAG GAG         195
TTT GGC AAC CAG TTC CAA AAG GCT GAA ACC ATC CCT GTC CTC         237
CAT GAG ATG ATC CAG CAG ATC TTC AAT CTC TTC AGC ACA AAG         279
GAC TCA TCT GCT GCT TGG GAT GAG ACC CTC CTA GAC AAA TTC         321
TA                                                              323
```

welches für die im IFN-α2(Arg) enthaltene Teilsequenz der Formel

| | | | | | | | | | | | | | | |
|---|---|---|---|---|---|---|---|---|---|---|---|---|---|---|
| | | | Leu | Ala | Gln | Met | Arg | Arg | Ile | Ser | Leu | Phe | Ser | 28 |
| Cys | Leu | Lys | Asp | Arg | Arg | Asp | Phe | Gly | Phe | Pro | Gln | Glu | Glu | 42 |
| Phe | Gly | Asn | Gln | Phe | Gln | Lys | Ala | Glu | Thr | Ile | Pro | Val | Leu | 56 |
| His | Glu | Met | Ile | Gln | Gln | Ile | Phe | Asn | Leu | Phe | Ser | Thr | Lys | 70 |
| Asp | Ser | Ser | Ala | Ala | Trp | Asp | Glu | Thr | Leu | Leu | Asp | Lys | Phe | 84 |

codiert. Dieses neue IFN-α2(Arg) unterscheidet sich von allen in Nature 287, 408–411 (1980) beschriebenen Interferonen in dem Bereich der Aminosäuren Nr. 18–84, nämlich durch Arg in den Positionen 23 und 34.

Die ausgewählten Zellen werden zweckmäßigerweise zum Zeitpunkt maximaler IFN-mRNA-Synthese denaturiert, zweckmäßigerweise 6–12 Stunden, vorzugsweise jedoch 9 Stunden, nach der Virusinduktion. Nach Abtrennen der Zellkerne wird aus dem Zellcytoplasma die RNA durch Phenolextraktion und Alkoholfällung gereinigt, nachfolgend durch Oligo (dT)-Zellulose-Chromatographie die Poly(A)$^+$RNA isoliert und diese durch Gradientenzentrifugation im Hinblick auf interferonspezifische Sequenzen angereichert (siehe linke Spalte der Figur 1).

Die so gereinigte Poly(A)$^+$RNA wird als Matrize zur Herstellung von einzelsträngiger cDNA mittels des Enzyms Reverse Transkriptase verwendet. Der zu diesem Einzel-DNA-Strang komplementäre zweite DNA-Strang wird mittels DNA-Polymerase I synthesiert. Die cDNA-Synthese und die Synthese des komplementären zweiten DNA-Stranges erfolgt in einer Lösung, welche die Deoxynucleosidtriphosphate des Adenosins, Thymidins, Guanosins und Cytidins enthält. Die erhaltene doppelsträngige cDNA-Mischung wird anschließend an beiden Strangenden durch das Enzym S1 Nuklease so modifiziert, daß überhängende Einzelstrangregionen entfernt werden, und jene doppelsträngigen DNA-Moleküle mittels Gradienten-Zentrifugation isoliert, welche mindestens 600 Basenpaare aufweisen. Die so erhaltene cDNA-Mischung wird mittels des Enzyms Terminale Transferase durch Oligodeoxycytidin-Anlagerung am 3′-Ende beider Stränge um etwa 15 Nukleotide verlängert, womit ein Bestandteil der Synthese rekombinater Moleküle fertiggestellt ist (siehe linke Spalte der Figur 1).

Als zweite Komponente wird ein zirkuläres doppelsträngiges Plasmid, vorzugsweise aus Escherichia coli stammend, z.B. Escherichia coli Plasmid pBR322, mittels Restriktionsendonuklease Pst I linearisiert und analog der cDNA-Mischung durch Anlagerung von Oligodeoxyguanidin an die 3′-Enden des Moleküls so modifiziert, daß überhängende Oligodeoxyguanidinenden entstehen. Diese Enden können mit den freien Oligodeoxycytidinenden der cDNA-Moleküle stabile DNA Doppelstrang-Regionen bilden (siehe rechte Spalte der Figur 1).

Mit den so hergestellten Hybridplasmiden, z.B. aus pBR322 und cDNA, wird ein Mikroorganismus als Wirt, z.B. Escherichia coli HB 101, transformiert, in dem die Replikation und Expression dieser DNA erfolgt.

Aus den so hergestellten Klonen werden nun diejenigen, welche für IFN-α oder für IFN-β spezifische Sequenzen enthalten, durch Koloniehybridisierung nachgewiesen. Als Probe dient radioaktiv markierte cDNA, welche durch reverse Transkription von IFN-mRNA-hältiger RNA, mit dem für Interferonsequenzen spezifischen Tridekanukleotid 5′dCCTTCTGGAACTG3′ als Primer, synthetisiert wird. Hierzu wird das Tridekanukleotid radioaktiv am Ende markiert, vorzugsweise mit $\gamma$–$^{32}$p–ATP und T4-Polynukleotid-Kinase. Die Herstellung des Initiationskomplexes für die Reverse-Transkriptase-Reaktion mit einem Überschuß des markierten Tridekanukleotids und Poly(A)$^+$RNA aus Sendaivirus-induzierten Zellen erfolgt analog den vorstehend beschriebenen Bedingungen (siehe Figur 2). Eine so hergestellte cDNA trägt also nur dann eine radioaktive Markierung, wenn der Initiationskomplex für die Reverse Transkriptase-Reaktion mit dem Tridekanukleotid stattgefunden hat, da ja die Markierung ausschließlich in diesem Segment enthalten ist. Hierdurch wird erfindungsgemäß eine hohe Selektivität für das Erkennen der DNA mit einer zum Tridekanukleotid komplementären DNA-Sequenz und damit für α- und β-Interferon-DNA gewährleistet. Das Sichtbarmachen jener transformierten Bakterienkolonien, welche Hybridplasmide mit zum eingesetzten Tridekanukleotid komplementären DNA-Sequenzen enthalten, erfolgt durch Autoradiographie (siehe Figur 3). Auf diese Weise wurden von etwa 13 000 transformierten Bakterienklonen 190 Klone isoliert, welche mit der durch das Tridekanukleotid initiierten cDNA ein positives Signal ergaben, das heißt, etwa 1% aller Klone der Genbank enthalten die spezifische Sequenz dCCTTCTGGAACTG.

Der Nachweis der interferonspezifischen Nukleinsäuresequenzen in den rekombinanten DNA-Molekülen erfolgt durch RNA-Transfer-Hybridisierung, Hybridisolierung, Restriktionskartierung und Nukleinsäuresequenzierung. Der biologische Nachweis der gebildeten Interferonpolypeptide erfolgt durch Bestimmung der antiviralen Aktivität, die Bestimmung des Interferontyps durch immunologische Methoden.

Das auf diese Weise isolierte IFN-α2(Arg)-Gen kann dann in Bakterien, aber auch in anderen Organismen, wie z.B. Hefe, zur Expression gebracht werden, wobei durch Vorschalten eines Promotors in Kombination mit einer Ribosomenbindungssequenz Werte bis zum etwa $10^4$-fachen der Spontanexpression erzielt werden können.

Die nachfolgenden Beispiele sollen die Erfindung näher erläutern:

Beispiel A

Auswahl einer geeigneten Zellinie zur Produktion von Poly(A)$^+$RNA, welche humane IFN-α und IFN-β-mRNA enthält:

Verschiedene humane Zellinien wurden mit Sendaivirus zur Interferonproduktion nach literaturbekannten Verfahren induziert und aus den Zellüberständen nach 24–28 Stunden der Gehalt an IFN-α und IFN-β durch Neutralisation mit typspezifischen Antiseren bestimmt. Die gefundenen Mengenverhältnisse zwischen IFN-α und IFN-β in einigen der getesteten Zellinien sind in der Tabelle auf Seite 2 zusammengestellt. Dabei zeigt sich, daß Namalwa Zellen z.B. etwas mehr als 50% IFN-α und bis zu etwa 50% IFN-β nach Sendaivirus Induktion produzieren.

Der Neutralisationstest wurde wie folgt durchgeführt:

Ungefähr 10 Interferon-Einheiten aus den Überständen der mit Sendaivirus induzierten Zellkulturen wurden 60–90 Minuten bei 37 °C inkubiert mit:

1. Antiserum gegen IFN-α; endgültige Verdünnung 1:100; (erhalten von Research Resources Branch, National Institute of Allergy and Infections Diseases, Bethesda Md, USA).
2. Antiserum gegen Human β-IFN; endgültige Verdünnung 1:300; (erhalten von Dr. Y.H. Tan, University of Calgary, Canada).
3. Mischung aus 1. und 2.

Nach der Inkubation wurde die Rest-Interferonaktivität im Plaquereduktionstest (siehe Journal of Interferon Research 2, 575–585 (1982)) bestimmt.

Beispiel B

Herstellung von Poly(A)$^+$RNA, welche menschliche IFN-α und IFN-β-mRNA enthält, aus Sendaivirus induzierten Namalwazellen

Züchtung der Namalwazellen und Induktion mit Sendaivirus erfolgte nach literaturbekannten Methoden (siehe Methods in Enzymology, Vol 78A, pp. 69–75 (1981), Academic Press, New York). Der Zeitpunkt der mRNA Präparation wurde mit 9 Stunden (6–12 Stunden) nach der Induktion mit Sendaivirus gewählt, da nach diesem Intervall der Anteil an interferonspezifischer mRNA ein Maximum erreicht.

Die Zellen wurden 20 Minuten bei 1000 g abzentrifugiert, einmal in NP-40-Puffer (0,14 M NaCol, 1,5 mM $MgCl_2$, 10 mM Tris-HCl, pH 7,4) gewaschen und in NP 40-Puffer mit 0,5% des nicht ionischen Detergens NP-40 (Shell) und 2 mg/ml Bentonit resuspendiert. Nach 5 Minuten im Eisbad wurden die Zellkerne durch Zentrifugation wie oben pelletiert, die (RNA-hältige) cytoplasmatische Fraktion (Überstand) wurde nach Zugabe von 2% SDS, 5 mM EDTA und 50 mM Tris-HCl, pH 9, 3 × mit Phenol-Chloroform und 1 × mit Chloroform extrahiert und die RNA anschließend alkoholgefällt. Poly(A)$^+$RNA wurde nach literaturkbekannter Methode (Proc. Natl. Acad. Sci USA, 69, 1408–1412 (1972)) durch Oligo-(dT)-Zellulose-Chromatographie gereinigt und durch Zentrifugation in einem 5–20% Saccharosegradienten in 10 mM Natriumacetatpuffer, pH 5,5, 1 mM EDTA (20 Stunden bei 25 000 U/min mit einem Spinco SW 27-Rotor) der Molekülgröße nach aufgetrennt und Moleküle der Größenordnung zwischen 6 S (Sedimentationseinheiten) und 18S (der Einfachheit halber «12S-RNA» genannt) gesammelt (siehe linke Seite der Figur 1).

Der Gehalt an Interferon-spezifischer mRNA (IFN-α und IFN-β-mRNA) wurde durch Mikroinjektion von «12S-RNA» in Xenopus laevis-Oocyten (siehe J. Embryol. and Exper. Morph. 20, 401–414 (1968)) und Messen der Interferonaktivität im Oocytenüberstand bestimmt. Dabei ergab 1 µg injizierter «12S-RNA» einen mittleren Interferontiter von etwa 1000 Internationalen Interferoneinheiten (I.E.) bezogen auf den Interferonstandard 69/19:

| Menge «12S»-Poly(A)$^+$RNA aus Sendaivirus-induzierten Namalwazellen | Einheiten Interferon pro ml Oocytenüberstand | Prozent Interferonaktivität neutralisiert durch Antiseren gegen: IFN-α | IFN-α + IFN-β |
|---|---|---|---|
| 1µg | 1000 | 80 | >98 |

Etwa 80% dieser Aktivität war also durch Antiserum gegen α-Typ Interferon neutralisierbar, während die gesamte Aktivität nur durch ein Gemisch aus Anti-α und Anti-β-Interferon-Antiserum neutralisierbar war.

Beispiel C

Herstellung einer Namalwa-cDNA-Klonbank

Die Poly(A)$^+$RNA, welche eine Molekülgröße zwischen 6–18S besitzt («12S-RNA») (siehe Beispiel B) wurde als Matrize zur Herstellung von einzelsträngiger cDNA verwendet, wobei 40 µg/ml Poly(A)$^+$RNA in 50 mM Tris-HCl, pH 8,3, 10 mM $MgCl_2$, 100 mM KCl, 1 mM dNTPs, 0,4 mM DTT, 4 mM Na-Pyrophosphat, 20 µg/ml Oligo(dT)$_{12-18}$ (PL-Biochemicals), 25 µg/ml Actinomycin D mit 100 Einheiten/ml AMV Reverse Transkriptase (Dr. J. Beard, Life Sciences, Inc. 1509$\frac{1}{2}$ 49$^{th}$ Street, South, St. Petersburg, Florida 33707, USA) 45 Minuten bei 44–45 °C inkubiert wurden. Anschließend wurde der RNA-Anteil des RNA-DNA-Hybrides durch einstündige Inkubation in 0,3 M NaOH bei 50 °C entfernt, danach die einzelsträngige cDNA neutralisiert und äthanolgefällt.

Der zum Einzel-DNA-Strang komplementäre zweite DNA-Strang wurde unter folgenden Bedingungen synthetisiert: 30 µg/ml einzelsträngige cDNA wurden in 0,12 M Kaliumphosphatpuffer, pH

6,9, 10 mM $MgCl_2$, 10 mM DTT, 1 mM dNTPs mit 300 Einheiten/ml DNA Polymerase I (Boehringer Mannheim) 6 Stunden bei 15 °C inkubiert, dann phenolextrahiert und äthanolgefällt.

Die so hergestellte doppelsträngige cDNA-Mischung wurde an den beiden Strangenden derart modifiziert, daß überhängende Einzelstrangregionen entfernt wurden. Dazu wurde die DNA in 0,3 M NaCl, 30 mM Natriumacetat, pH 4,5, 1 mM $ZnCl_2$ mit 1250 Einheiten/ml S1-Nuklease (Miles Laboratories) 30 Minuten bei 37 °C inkubiert, hierauf wurde phenolextrahiert und äthanolgefällt. Die so hergestellte «blunt ended» doppelsträngige cDNA wurde auf einem 5–20% Saccharosegradienten in 10 mM Natriumacetatpuffer, pH 5,5, 1 mM EDTA, aufgetrennt und jene Fraktionen isoliert, welche eine Länge von mindestens 600 Basenpaaren aufwiesen.

Von dieser cDNA-Mischung wurden 0,5 µg durch Oligodeoxycytidin-Anlagerung am 3'Ende beider Stränge, durch Inkubation in 140 mM Kaliumcacodylat, pH 6,9, 30 mM Tris-HCl, pH 6,9, 2 mM $CoCl_2$, 0,1 mM DTT, 0,1 mg/ml BSA, 5 µM dCTP mit 500 Einheiten/ml Terminaler Transferase, 4 Minten bei 37 °C, um etwa 15 Nukleotide verlängert. Mit diesem Schritt ist die cDNA-Mischung, als ein Bestandteil der rekombinanten Moleküle, fertiggestellt (siehe linke Seite Fig. 1).

Als zweite Komponente wurde das Escherichia coli-Plasmid pBR322, ein zirkuläres doppelsträngiges DNA Molekül, herangezogen. Ein Aliquot von 2 µg pBR322 wurde durch die Restriktionsendonuklease Pst I linearisiert und in ähnlicher Weise wie die cDNA-Mischung durch Anlagerung von Oligodeoxyguanidin an die 3' Enden des Moleküls derart modifiziert, daß überhängende Oligodeoxyguanidinenden erzeugt wurden (rechte Seit der Figur 1). Diese Oligodeoxyguanidinenden können mittels Basenpaarung mit den freien Oligodeoxycytidinenden der cDNA Moleküle stabile DNA Doppelstrang-Regionen erzeugen. Dazu werden die beiden Komponenten dieser Reaktion in 0,1 M NaCl, 1 mM EDTA, 20 mM Tris-HCl, pH 8, 10 Minuten bei 65 °C, dann 2,5 Stunden bei 45 °C, dann über Nacht bei 37 °C inkubiert.

Durch diese Methode wird die Pst I Restriktionsendonuklease-Spaltstelle regeneriert und kann in der Folge benützt werden, um die cDNA-Inserte aus dem Vektorhybrid herauszuschneiden.

Die nach dieser Methode hergestellten Hybridplasmide aus pBR322 und Namalwa-cDNA wurden zur Transformation von Escherichia coli HB 101 nach literaturbekannter Methode (Proc. Natl. Acad. Sci, USA 69, 2110–2114 (1972)) verwendet. Mit dieser Methode wurden 30 000 Klone transformierter E. coli Zellen erhalten und einzeln in Mikrotiterplatten aufgeteilt.

Beispiel D

Herstellung einer bezüglich IFN-α und IFN-β-DNA Sequenzen spezifischen Hybridisierungsprobe unter Benutzung eines synthetischen Tridekanukleotids 5'dCCTTCTGGAACTG3'

Die angewendete Selektionsmethode für transformierte E. coli Klone, welche Interferonsequenzen enthalten, basiert auf der Verwendung eines synthetischen Tridekanukleotids mit der Sequenz: 5'dCCTTCTGGAACTG3'. Diese Sequenz stellt das längste, nicht unterbrochene DNA Segment dar, in welchem Homologie zwischen den meisten IFN-α Genen und dem IFN-β Gen vorliegt. Die Synthese des Tridekanukleotids wurde bereits beschrieben (siehe European Journal of Cell Biology, *loc. cit.*). Das Tridekanukleotid wurde in 50 mM Tris-HCl, pH 7,5, 10 mM $MgCl_2$, 5 mM DTT, 0,1 mM Spermidin, 1 µM $^{32}P$-γ-ATP mit 60 Einheiten/ml T4-Polynukleotidkinase (Bethesda Research Laboratories) 30 Minuten bei 37 °C zu einer spezifischen Aktivität von etwa 500 Ci/mMol am 5' Ende des Moleküls markiert.

Dieses endmarkierte Tridekanukleotid wurde als Primer für (einzelsträngige) cDNA-Synthese verwendet, wobei als Matrize Poly(A)$^+$RNA aus Sendaivirus-induzierten Namalwazellen (siehe Beispiel B) diente. Die Reaktion wurde bei einem 5–10 fachen molaren Überschuß von Primer gegenüber RNA in 50 mM Tris-HCl, pH 8,3, 60 mM KCl, 5 mM DTT, 50 µg/ml Actinomycin D, 4 mM $MgCl_2$, 4 mM Natriumpyrophosphat, 0,5 mM dNTPs mit 100 Einheiten/ml AMV Reverse Transkriptase 90 Minuten bei 37 °C durchgeführt. Nach Entfernung des RNA-Anteiles des RNA-DNA-Hybrides durch einstündige Inkubation in 0,3 M NaOH bei 50 °C und anschließende Neutralisation mit 0,3 M Essigsäure ist die cDNA als Hybridisierungsprobe verwendbar. Eine schematische Darstellung dieses Verfahrens ist in Figur 2 wiedergegeben. Eine so hergestellte cDNA trägt demnach die radioaktive Markierung nur in Molekülen, die vom interferonspezifischen Tridekanukleotid als Primer ausgehend synthetisiert worden sind und zeigt somit eine hohe Spezifität für das Erkennen von Interferon-DNA-Sequenzen in Hybridisierungen.

Beispiel E

Koloniehybridisierung mit Tridekanukleotid-geprimter cDNA

Die hier dargestellte Methode dient der Erkennung jener transformierten Bakterienklone, welche Hybridplasmide mit zum Tridekanukleotid komplementären DNA Sequenzen enthalten. Dazu wurden Zellen von einzelnen geklonten Transformanten auf 22 × 15 cm Nitrozellulosefilter (Porengröße 0,45 µm, Millipore oder Schleicher & Schuell) transferiert, zu einer Koloniegröße von 2 mm Durchmesser auf den Filtern gezüchtet und nach literaturbekannter Methode für die Koloniehybridisierung vorbereitet (Proc. Natl. Acad. Sci USA 72, 3961–3965 (1975) und Analytical Biochemistry 98, 60–63 (1979)). Die Nukleinsäurehybridisierung mit endmarkierter cDNA (siehe Beispiel D) wurde in 50% Formamid, 4 × SET (1 × SET = 0,15 M NaCl, 5 mM EDTA, 50 mM Tris-HCl, pH 8), 1 × Denhardt's Lösung (0,02% BSA, 0,02% Ficoll, 0,02% Polyvinylpyrrolidon), 0,5 mg/ml denaturierter und gescherter Lachssperma DNA, 0,1% Natriumpyrophosphat und 0,1% SDS für 48 Stunden bei 37 °C durchgeführt. Die Filter wurden hierauf in einer Lösung bestehend aus 50% Formamid und

0,2 × SSC (1 × SSC = 0,15 M NaCl, 0,015 M Natriumcitrat) bei 20–25 °C mehrere Stunden (6–8 Stunden) gewaschen und dann mit einer Lösung aus 1 × SSC gespült. Die Filter wurden bei 20–25 °C getrocknet und bei −70 °C mit einem Röntgenfilm (Kodak XR) exponiert. Die Figur 3 zeigt ein typisches Ergebnis eines derartigen Versuches: Auf der abgebildeten Fläche wurden 1536 Bakterienkolonien gezüchtet und durch Koloniehybridisierung gescreent. Der Pfeil zeigt auf ein Beispiel eines transformierten Bakterienklons, dessen Hybridplasmid-DNA mit der radioaktiven Probe ein Signal ergibt. Insgesamt wurden durch Screenen von etwa 13 000 transformierten Bakterienkolonien 190 Klone isoliert, welche mit der durch das Tridekanukleotid initiierten cDNA aus Sendaivirus-induzierten Namalwazellen ein positives Signal ergaben; sie wurden auf 2 Mikrotiter-Platten (P1 und P2) gesammelt. Demnach enthielten etwa 1% aller Klone der Klonbank die spezifische Sequenz dCCTTCTGGAACTG.

Beispiel F

Analyse der Sequenzkomplexität der mit Tridekanukleotid-geprimter cDNA hybridisierenden Klone

Um die Anzahl der Klone mit verschiedener Sequenz (Sequenzkomplexität) in den 190 Tridekanukleotid-positiven Klonen (siehe Beispiel E) festzustellen, wurden von verschiedenen Klonen die cDNA-Inserte durch Pst I-Verdauung (siehe Beispiel C), Elektrophorese in 0,8% Agarosegelen und Elution der gewünschten Bande (durch Ausscheiden des die Bande beinhaltenden Gelstückchens und elektrophoretische Elution der DNA aus der Agarose in einen Dialysenschlauch) isoliert und nach literarubekannter Methode durch Nick-Translation mit $^{32}P$ markiert (J. Mol. Biol. 113, 237–251 (1977)). Abdrücke der Tridekanukleotid-positiven Klone (Mikrotiterplatten P1 und P2) und einer willkürlich ausgewählten Mikrotiterplatte (E52) wurden auf Nitrozellulosefilter transferiert, aufgezüchtet, für Koloniehybridisierung vorbereitet (siehe Beispiel E) und mit den verschiedenen $^{32}P$ markierten cDNA-Inserten hybridisiert. Die Hybridisierungen wurden durchgeführt wie in Beispiel E beschrieben, nur daß in die Hybridisierungslösung auch 50 µg/ml poly(U) und 10 µg/ml Poly(I) : poly (C) zugegeben wurden und die Filter nach der Hybridisierung in 50% Formamid und 1 × SSC gewaschen wurden. Klon PIA6 wurde auf diese Weise analysiert und hybridisierte jeweils nur mit sich selbst.

Beispiel G

RNA-Transfer-Hybridisierungen

Um herauszufinden ob ein bestimmter Tridekanukleotid-positiver Bakterienklon Interferonsequenzen enthalten könnte, wurde getestet, ob seine Plasmid-DNA mit einer in Namalwazellen durch Sendaivirus induzierten mRNA hybridisiert. Dazu wurde Poly(A)$^+$-RNA von induzierten und nicht induzierten Namalwazellen analog der in Beispiel B angegebenen Weise isoliert, durch Inkubation eine Stunde bei 50 °C in 1 M Glyoxal, 50% DMSO, 10 mM Natriumphosphatpuffer, pH 7 denaturiert, auf einem 1,4% Agarosegel aufgetrennt, nach literaturbekannter Methode (Proc. Natl. Acad. Sci. USA 77, 5201–5205 (1980)) auf Nitrozellulosefilter transferiert und mit durch Nick-Translation $^{32}P$ markierter (siehe Beispiel F) Plasmid-DNA hybridisiert. Plasmid-DNA, die von einem induzierten Gen abstammt, wird in dieser Versuchsanordnung nur mit RNA aus induzierten Zellen hybridisieren, nicht aber mit RNA aus uninduzierten Zellen. Figur 4 zeigt die Autoradiographie eines solchen Experiments: induzierte RNA («i») ist in der linken Spalte, nicht induzierte RNA («n») in der rechten Spalte. Die zur Hybridisierung verwendete Plasmid-DNA P1A6 hybridisiert nur mit RNA aus induzierten Zellen. Die Molekülgröße der mit P1A6 hybridisierenden RNA liegt bei 12–14S. Das Plasmid P1A6 wurde daher weiter untersucht, um seinen Gehalt an Interferon-DNA-Sequenz eindeutig festzustellen.

Beispiel H

Analyse des IFN-α-Typ Klons P1A6

Der Klon P1A6 hybridisiert mit virus-induzierter Namalwa-RNA in der Region von 12–14S (beschrieben in Beispiel G). Er enthält ein cDNA-Insert von etwa 900 Basenpaaren, das, wie Restriktionsanalyse ergab, keine Spaltstellen für die Enzyme Bam HI, Bgl II, Eco RI, Hin dIII, Pst I und Pvu II aufweist (siehe Figur 5).

Der Beweis dafür, daß Klon P1A6 IFN-α-Sequenzen beinhaltet, wurde wieder durch DNA-Sequenzanalyse erbracht: die Sequenz des cDNA-Inserts von P1A6 wurde von den das Insert begrenzenden PsT I-Spaltstellen nach innen ermittelt (siehe Figur 8) und mit den von Goeddel et al. (Nature 290, 20–26 (1981)) beschriebenen IFN-α-Sequenzen verglichen. Es zeigt sich, daß Klon P1A6 eine um 49 Basenpaare kürzere Variante von LeIFN C darstellt. Das Vorhandensein von Poly(A)-Sequenzen am 3′-Ende von P1A6 weist darauf hin, daß Klon P1A6 von einer kürzeren mRNA abstammt als der publizierte Klon LeIFN C. Die für LeIFN C-Protein codierende Region ist jedoch vollständig vorhanden.

Beispiel I

Identifikation und Analyse eines weiteren IFN-α-Typ Klons (1F7)

Klon P1A6 war der einzige IFN-α-Typ-Klon, der aus der Kollektion der 190 Tridekanukleotid-positiven Klone (siehe Beispiel E) identifiziert werden konnte. Um weitere IFN-α-Typ-Klone zu ermitteln, wurden 1800 Klone der ursprünglichen cDNA-Klonbank (Beispiel C) durch Koloniehybridisierung (wie in Beispiel F beschrieben) mit dem cDNA-Insert des Klons P1A6 hybridisiert. Ein weiterer IFN-α-Klon (Klon 1F7) wurde auf diese Weise gefunden. Restriktionsanalyse und teilweise DNA-Sequenzierung des cDNA Inserts von 1F7 ergaben, daß es sich bei 1F7 um eine um 175 Basenpaare längere Variante des Klontyps Le IFN A (Goeddel et al., siehe oben) handelt. 1F7 enthält wie LeIFN A je 2 Spaltstellen für die Enzyme Bgl II und Pvu II (Figur 5); die ermittelten DNA-Sequenzen am 5'- und am 3'-Ende des Inserts sind in Figur 6 angegeben.

Desweiteren wurden Teile der DNA-Sequenz des IFN-α-Typ Klons (1F7) nach der Dideoxy-Methode (siehe Messing et al. in Nuc. Acid. Res. 9, 309 (1981) und Gardner, R.C. et al. in Nuc. Acid. Res. 9, 2871 (1981)) mit einem Universal-Primer analysiert. Für die Positionen 120 bis 327 wurde folgende Teilsequenz gefunden:

......CCTGGCACAGATGAGGAGAATCTCTCTTTTCTCCTGCTTGAAGGACAGACG
TGACTTTGGATTTCCCCAGGAGGAGTTTGGCAACCAGTTCCAAAAGGCTGAAACCA
TCCCTGTCCTCCATGAGATGATCCAGCAGATCTTCAATCTCTTCAGCACAAAGGAC
TCATCTGCTGCTTGGGATGAGACCCTCCTAGACAAATTCTA.....

Im Vergleich zu der literaturbekannten Sequenz des IFN-αA Typs (siehe D.V. Goeddel et al. in Nature 290, 20 (1981)) weist die neue DNA-Sequenz des IFN-α-Typ Klons (1F7) im Bereich der Nukleotide Nr. 120–327 folgende Unterschiede auf: In Position 137 und 170 wurde jeweils das Nukleotid A durch G ersetzt.

Beispiel J
Interferonaktivität in Lysaten von E. coli HB101, transformiert mit dem hybriden Plasmid 1F7

Lysate von mit P1A6 oder 1F7 transformierten Bakterienkulturen wurden auf ihren Gehalt an biologisch aktivem Interferon untersucht. Dazu wurden 100 ml Bakterienkultur in L-Broth-Medium zu einer optischen Dichte von 0,6–0,8 gezüchtet, durch Zentrifugation 10 Minuten bei 7000 U/min pelletiert, 1 × in 50 mM Tris-HCl, pH 8, 30 mM NaCl gewaschen und schließlich in 1,5 ml desselben Puffers suspendiert. Nach Inkubation mit 1 mg/ml Lysozym 30 Minuten auf Eis wurden die Bakterien 5 × gefroren und getaut und hierauf die Zellbruckstücke durch Zentrifugation eine Stunde bei 40 000 U/min entfernt. Der Überstand wurde steril filtriert und im Plaquereduktionstest auf Interferonaktivität getestet. In auf diese Weise gewonnenen Lysaten von Klon 1F7 konnten etwa 500 Einheiten pro ml Interferon nachgewiesen werden. Klon 1A6 ergab in diesem Test keine Aktivität, was vermutlich auf eine andere Orientierung des Inserts im Plasmidvektor zurückgeführt werden kann.

Beispiel K
Verbesserung der Expression des von 1F7 codierten Interferons

Um gute Expression eines Genes zu erreichen, müssen auf DNA-Ebene drei Voraussetzungen erfüllt werden: erstens muß vor dem Gen ein Promotor (eine RNA-Polymerase-Bindungsstelle) liegen, zweitens muß die abgelesene RNA vor dem Initiationscodon der Translation eine Ribosomenbindungssequenz (RBS) tragen und drittens muß der Abstand zwischen RBS und Initiationscodon eine geeignete Länge haben.

Zur Expression des von 1F7 codierten Interferons wurde als Promotorsequenz der literaturbekannte Promotor des Trytophanoperons von Serratia marcescens (Nature 276, 684–689 (1978)) genommen und als RBS eine literaturbekannte DNA-Sequenz (Proc. Natl. Acad. Sci USA 78, 5543–5548 (1981)). Zur Ermittlung des optimalen Abstandes zwischen der RBS und dem Initiationscodon von 1F7 wurde folgende Strategie gewählt: die 1F7-cDNA-Sequenz wurde mit der für doppelsträngige DNA spezifischen Exonuklease BAL 31 kurz verdaut, wodurch ein Gemisch von Molekülen verschiedener Länge erhalten wurde. Diese Moleküle wurden hierauf mit Hilfe von synthetischen Linkersequenzen in geeigneter Weise in ein «Expressionsplasmid» ligiert, das heißt, in ein Plasmid, das Promotor und RBS enthielt. E. coli HB101 wurde mit diesen Plasmiden transformiert und einzelne Transformanten wurden auf Interferonsproduktion getestet.

Im Detail wurde der Versuch folgendermaßen durchgeführt: 0,5–1 µg 1F7-Insert wurden in 100 µl 20 mM Tris-HCl, pH 8,1, 0,6 M NaCl, 12 mM $MgCl_2$, 12 mM $CaCl_2$, 1 mM EDTA 3 min bei 30 °C mit 0,5 Einheiten BAL-31 (Bethesda Research Laboratories) inkubiert. Hierauf wurde die Reaktion durch Zugabe von 300 µl 15 mM EDTA, pH 7,8, beendet, es wurde 10 min auf 65 °C erwärmt, mit Phenol und Äther extrahiert und äthanolgefällt. Der Niederschlag wurde in 10 µl 70 mM Tris-HCl, pH 7,5, 7 mM $MgCl_2$, 1 mM DTT, 0,4 mM ATP, zusammen mit 30–60 pMol phosphorylierten Hin dIII-Linkern (Bethesda Research Laboratories) aufgenommen und mit 0,5–1 Einheiten T4-Ligase (Bethesda Research Laboratories) über Nacht bei 14 °C inkubiert. Die Reaktion wurde durch Erhitzen 10 min auf 65 °C beendet, 2 M $NH_4$-Acetat zugegeben und die Interferongene von nicht ligierten Linkern durch Fällung mit 0,6 Volumen Isopropanol gereinigt. Die Fällung wurde in 30–50 µl 33 mM Tris-Acetat, pH 7,9, 66 mM K-Acetat, 10 mM Mg-Acetat, 100 µg/ml BSA gelöst und mit 30–50 Einheiten Hin dIII 2–3 Stunden lang verdaut. Die Reaktion wurde durch Erhitzen 10 min auf 65 °C beendet und nach Zugabe von 2 M $NH_4$-Acetat wurden die Interferongene mit 0,6 Volumen Isopropanol gefällt. Der Niederschlag wurde in 10 µl 70 mM Tris-HCl, pH 7,5, 7 mM $MgCl_2$, 1 mM DTT, 0,5 mM ATP aufgelöst und mit Hilfe von $10^{-2}$ Einheiten T4-Ligase mit 0,2 µg Hin dIII-linearisiertem, phosphatasebehandeltem Expressionsplasmid pER103 über Nacht bei 14 °C ligiert. pER103 ist ein pBR322-Derivat, das die Promotorsequenz des Tryptophanoperons von S. marcescens und eine literaturbekannte RBS enthält (siehe oben); es kann mit Hin dIII in der Nähe der RBS linearisiert werden. Die so erhaltenen Plasmide wurden zur Transformation von E. coli HB101 verwendet und die Interferonproduktion einzelner Transformanten wurde auf die in Beispiel L beschriebene Weise getestet. Es ergab sich eine Steigerung der Interferonexpression bis zum etwa $10^4$-fachen Wert der Spontanexpression.

Folgende Mikroorganismen und rekombinante-DNA-Moleküle wurden bei der Hinterlegungsstelle «Deutsche Sammlung von Mikro-Organismen, Grisebachstraße 8, 3400 Göttingen», am 17. Mai 1982 unter den DSM-Nummern

2362 (1F7) und 2363 (P1A6)

hinterlegt und sind der Öffentlichkeit gemäß Budapester Vertrag zugänglich.

| Verwendete Begriffe und Abkürzungen | |
|---|---|
| (d)A: | (Deoxy) Adenosin |
| Antiserum: | ein Serum, das Antikörper beinhaltet |
| ATP: | Adenosintriphosphat |
| Autoradiographie: | fotographische Methode zum Nachweis von Radioaktivität |
| Basenpaar: | 2 komplementäre Nukleotide, z.B. A-T, G-C |
| blunt ends: | vollständig basengepaarte Enden eines DNA-Doppelstrangmoleküls, zum Unterschied von überhängeden Einzelstrang-Enden |
| BSA: | Bovinserumalbumin |
| (d)C: | (Deoxy) Cytidin |
| cDNA: | eine zu RNA komplementäre DNA |
| cDNA-Pool: | Gemisch aus cDNAs verschiedener Sequenz |
| codieren: | die Information für etwas tragen; DNA trägt in der Nukleotidsequenz die Information für die Aminosäuresequenz eines Proteins |
| DMSO: | Dimethylsulfoxid |
| DNA: | Deoxyribonukleinsäure |
| DNA-Sequenz: | eine lineare Anordnung von Nukleotiden, welche durch Phosphodiesterbindungen verknüpft sind. |
| DTT: | Dithiothreitol |
| EDTA: | Äthylendinitrilotetraessigsäure |
| Elektrophorese: | Trennung von (DNA oder RNA-) Molekülen nach Molekülgrösse im elektrischen Feld |
| Expression: | Umsetzung der Information eines Gens in mRNA durch Transkription und in weiterer Folge in Polypeptid (Protein) durch Translation |
| Filterhybridisierung: | Hybridisierung von Nukleinsäuren, wobei ein Partner filtergebunden ist |
| (d)G: | (Deoxy) Guanosin |
| Gen: | Abschnitt auf der DNA, der die Information für ein bestimmtes Transkript (RNA-Molekül) trägt, das in weiterer Folge in ein Protein übersetzt werden kann. |
| Genfamilien: | Familien von Genen, die für nahe verwandte Produkte (Subtypen) codieren |
| Genprodukt: | RNA (Transkript), Protein (Translationsprodukt) |
| Gradient: | siehe Saccharosegradient |
| Homologie (von DNA Sequenzen): | Verwandtschaft zwischen DNA-Sequenzen, die sich in einer ähnlichen Nukleotidabfolge zeigt |
| Hybrid: | stabiler Komplex von zwei zueinander zumindest zum Teil komplementären DNA-Strängen |
| Hybridisierung (von Nukleinsäuren): | Ausbildung von stabilen Komplexen zwischen zueinander komplementären einzelnen DNA- oder RNA-Strängen |
| Hybridisierungsprobe: | radioaktiv markierte Nukleinsäure, die zur Auffindung von dazu komplementären Sequenzen dient |
| Hybridplasmid: | Plasmid, das einen DNA-Abschnitt aus einem fremden Organismus enthält |
| I: | Inosin |
| IFN-α: | Leukocyteninterferon |
| IFN-β: | Fibroblasteninterferon |
| IFN-γ: | Immuninterferon |
| in vitro: | im Reaktionsglas |
| Induktion (von Interferonproduktion): | Stimulation von Zellen zu Synthese von Interferon durch Behandlung mit Induktoren (Viren, doppelsträngige RNA, Mitogene |

| Verwendete Begriffe und Abkürzungen | |
|---|---|
| Initiationscodon: | Sequenz AUG der mRNA, die den Beginn der Translation signalisiert |
| Initiationskomplex: | Verbindung von Reverser Transkriptase mit mRNA und Primer, die den Beginn der cDNA-Synthese ermöglicht |
| Insert, cDNA-Insert: | das Stück fremder DNA (z.B. eine cDNA), das sich in einem Hybridplasmid befindet |
| Klon: | Bakterienkolonie, von einem einzelnen Bakterium abstammend |
| Klonbank, cDNA-Klonbank: | eine Sammlung von Bakterienklonen, die alle ein Hybridplasmid mit einem cDNA-Insert beinhalten |
| Klonieren: | das Herstellen von Klonen; zumeist verstanden als das Herstellen von Klonen, die ein Hybridplasmid beinhalten |
| Koloniehybridisierung: | Hybridisierung mit filtergebundenen denaturierten Bakterienkolonien |
| komplementär: | zueinander passend (Nukleotide in der DNA: A ist komplementär zu T, G ist komplementär zu C) |
| Kreuzhybridisierung: | Hybridisierung zwischen nicht identischen, aber homologen DNA-Sequenzen |
| ligieren: | kovalentes Verknüpfen von DNA-Sequenzen mit Hilfe des Enzyms Ligase |
| Mikrotiterplatte: | Platte mit 96 Vertiefungen, die durch die Koordinanten 1-12 und A-Z charakterisiert sind |
| Mitogen: | Substanz, die Zellen zur Mitose (Zellteilung) stimuliert |
| molekulares Klonieren: | siehe Klonieren |
| mRNA: | Messenger RNA, ist eine für Proteine codierende RNA |
| Neutralisation: | Inaktivierung eines Antigens durch Antikörper |
| dNTPs: | Mischung der 4 Nukleotide dATP, dTTP, dCTG, dGTP |
| Nukleotide: | Bausteine einer DNA oder RNA; (d)A, (d)C, (d)G, (d)T |
| Oligonukleotide: | wenige miteinander durch Phosphodiesterbindungen verknüpfte Nukleotide |
| Oligo(C): | Oligomeres von C-Resten, durch Phosphodiesterbindungen verknüpft |
| Oligo(dT): | Oligomeres von dT-Resten, durch Phosphodiesterbindungen verknüpft |
| Oligo(dT)-Zellulose: | an Zellulose gebundene Oligo(dT)-Reste, zur Chromatographie von Poly(A)$^+$RNA |
| PIPES: | Piperazin-N, N'-bis(2-äthansulfonsäure) |
| Plasmid: | zirkuläre, extrachromosomale Bakterien-DNA, die selbständig repliziert |
| Poly(A) oder (dA): | Polymeres von A- oder dA-Resten, durch Phosphodiesterbindungen verknüpft |
| Poly(A)$^+$RNA: | mRNA, welche am 3'Ende der Nukleinsäuresequenz eine homopolymere Region von Poly(A) besitzt |
| Poly(C) oder (dC): | Polymeres von C- oder dC-Resten, durch Phosphodiesterbindungen verknüpft |
| Poly(I): | Polymeres von I-Resten, durch Phosphodiesterbindungen verknüpft |
| Poly(I):poly(C): | doppelsträngige Nukleinsäure, deren Stränge Poly(I) und Poly(C) sind |
| Poly(U): | Polymeres von U-Resten, durch Phosphodiesterbindungen verknüpft |
| Primer: | Oligonukleotid, komplementär zu einem Teil eines RNA-Moleküls, von dem ausgehend cDNA-Synthese erfolgt |
| Probe: | siehe Hybridisierungsprobe |
| Promotor: | DNA-Sequenz, an die RNA-Polymere bindet |
| RBS: | siehe Ribosomenbindungenssequenz |
| rekombinante DNA: | DNA-Sequenz, welche aus DNA-Abschnitten verschiedener Herkunft besteht, die in vitro miteinander verknüpft worden sind |
| Replikation (von DNA): | Duplizierung eines DNA-Moleküles |
| Restriktionsanalyse: | Kartierung eines DNA-Moleküls im Hinblick auf Spaltstellen von Restriktionsendonukleasen |

| Verwendete Begriffe und Abkürzungen | |
|---|---|
| Restriktionsendonuklease: | Enzym, das bei einer bestimmten DNA-Sequenz den DNA-Doppelstrang spaltet |
| Restriktionskartierung: | siehe Restriktionsanalyse |
| Reverse Transkription: | Herstellung einer cDNA-Kopie mit einem RNA-Molekül als Matrize und einem Oligonukleotid als Primer |
| Ribosomenbindungssequenz: | Teil der mRNA, der ans Ribosom binden kann |
| RNA: | Ribonukleinsäure |
| RNA-Polymerase: | Enzym, das einen zu DNA komplementären RNA-Strang synthetisieren kann |
| Saccharosegradient: | Mittel zur Auftrennung eines Gemisches von (RNA-) Molekülen nach ihrer Grösse |
| SDS: | Natriumdodecylsulfat |
| Screening: | Durchsuchen im Hinblick auf eine bestimmte Eigenschaft |
| Sequenzkomplexität: | ein Wert für die Anzahl qualitativ verschiedener DNA-Sequenzen in einer bestimmten Menge DNA |
| Subtypen: | siehe Genfamilien |
| (d)T: | (Deoxy) Thymidin |
| Transformant: | Bakterium, das fremde DNA durch Transformation erhalten hat |
| Transformation: | Einschleusen fremder DNA in Bakterien |
| Transkription: | Kopierung von mRNA von einer dazu komplementären DNA-Sequenz |
| Translation: | Umsetzung der Information vom mRNA in ein Polypeptid (Translationsprodukt) |
| Tridekanukleotid: | Oligonukleotid mit 13 Nukleotidbestandteilen |
| Tridekanukleotid-positiv: | mit dem Tridekanukleotid hybridisierend |
| Tris: | Trishydroxymethylaminomethan |
| U: | Uridin |
| Vektor: | Vehikel zum Einschleusen fremder DNA in Bakterien, meist ein Plasmid |
| Vektorhybrid: | siehe Hybridplasmid |

Kurze Beschreibung der Illustrationen:

Fig. 1:
ist eine schematische Darstellung der Herstellung einer cDNA-Klonbank.

Fig. 2:
ist eine schematische Darstellung der Herstellung einer cDNA als Hybridisierungsprobe, die bezüglich ihres Gehalts an IFN-α und IFN-β-spezifischen DNA Sequenzen durch Verwendung eines spezifischen Tridekanukleotid-Primers angereichert ist.

Fig. 3:
zeigt das Ergebnis der Koloniehybridisierung einer Klonbank mit Tridekanukleotid-initiierter cDNA aus Namalwazellen. Die Darstellung zeigt eine Autoradiographie eines Nitrozellulosefilters, welches 1536 verschiedene Klone trägt. Der Pfeil zeigt auf ein Beispiel eines Tridekanukleotid-positiven Klons.

Fig. 4:
zeigt die Autoradiographie einer Filterhybridisierung von Poly(A)$^+$RNA aus induzierten und nicht induzierten Namalwazellen mit Plasmid P1A6. Die virusinduzierte Poly(A)$^+$RNA («i» in der linken Spalte) und die Poly(A)$^+$RNA aus nicht induzierten Namalwazellen («n» in der rechten Spalte) wurden durch Elektrophorese nach Molekülgröße aufgetrennt, bevor der Transfer auf Nitrozellulosefilter, die Habridisierung der Filter mit radioaktiv markierter Plasmid-DNA und anschließende Autoradiographie erfolgten.

Fig. 5:
zeigt den schematischen Vergleich der Restriktionskarten von zwei DNA-Inserten aus rekombinanten DNA-Molekülen dieser Erfindung. Die DNA-Inserte der Plasmide P1A6 und 1F7 stellen für zwei unterschiedliche Subtypen von IFN-α codierende DNA-Sequenzen dar.

Fig. 6:
zeigt die Nukleotidsequenzen der 3′ und 5′ terminalen Segmente von 2 Inserten mit IFN-α-DNA-Spezifität (Klone P1A6 und 1F7). Die unterstrichene Sequenz ATG ist das Initiationscodon für die Translation.

**Patentansprüche**

1. Ein für IFN-α2(Arg) codierendes Polydeoxyribonukleotid, dadurch gekennzeichnet, daß dieses Polydeoxyribonukleotid einer im PstI-Insert des als pBR322(Pst)1F7 bezeichneten Plasmids, welches in E. coli HB101 bei der Deutschen Sammlung für Mikroorganismen unter der DSM-Nummer 2362 hinterlegt ist, vorliegenden Sequenz entspricht.

2. Eine DNA-Sequenz gemäß Anspruch 1, dadurch gekennzeichnet, daß die für IFN-α2(Arg) codierende Sequenz die Teilsequenz der Formel

| | | | | | | | | | | | | | | |
|---|---|---|---|---|---|---|---|---|---|---|---|---|---|---|
| | | 120 | | | | | | | | | | | | |
| | | C | CTG | GCA | CAG | ATG | AGG | AGA | ATC | TCT | CTT | TTC | TCC | 153 |
| TGC | TTG | AAG | GAC | AGA | CGT | GAC | TTT | GGA | TTT | CCC | CAG | GAG | GAG | 195 |
| TTT | GGC | AAC | CAG | TTC | CAA | AAG | GCT | GAA | ACC | ATC | CCT | GTC | CTC | 237 |
| CAT | GAG | ATG | ATC | CAG | CAG | ATC | TTC | AAT | CTC | TTC | AGC | ACA | AAG | 279 |
| GAC | TCA | TCT | GCT | GCT | TGG | GAT | GAG | ACC | CTC | CTA | GAC | AAA | TTC | 321 |
| TA | | | | | | | | | | | | | | 323 |

enthält.

3. Ein pBR322-Plasmid, enthaltend im PstI-Insert eine DNA-Sequenz gemäß Anspruch 1 oder 2.

4. Das als pBR322(Pst)1F7 bezeichnete Plasmid gemäß Anspruch 3, welches in E. coli HB101 bei der Deutschen Sammlung für Mikroorganismen unter der DSM-Nummer 2362 hinterlegt ist.

5. Verfahren zur Herstellung eines Klons enthaltend ein Plasmid gemäß Anspruch 3, dadurch gekennzeichnet, daß man aus cDNA, die mit RNA aus dem Sendai-Virus induzierten Namalwa-Zellen synthetisiert worden war, eine entsprechende Klonbank herstellt und aus dieser

a) mit Hilfe des Tridekanukleotids der Formel

5′-dCCTTCGGAACTG-3′

durch Hybridisierung einen Klon, der dieses Tridekanukleotid enthält und für LeIFN-B, C, D, F oder G codiert, ermittelt und

b) anschließend einen für IFN-α2(Arg) codierenden Klon mittels Hybridisierung mit dem cDNA-Insert eines gemäß a) erhaltenen Klons auswählt.

6. Ein Mikroorganismus enthaltend in einem Plasmid ein für IFN-α2(Arg) codierendes Polydeoxyribonukleotid gemäß Anspruch 1 oder 2.

7. Ein Mikroorganismus gemäß Anspruch 6 transformiert mit einem Plasmid gemäß Anspruch 3 oder 4.

8. E. coli transformiert mit einem Plasmid gemäß Anspruch 3 oder 4.

9. Das als IFN-α2(Arg) bezeichnete α-Interferon, das durch ein Polydeoxyribonukleotid gemäß Anspruch 1 oder 2 codiert wird, und im wesentlichen frei von anderem zellulärem Material ist.

10. IFN-α2(Arg) gemäß Anspruch 9, enthaltend in dem Bereich der Aminosäuren Nr. 18–84 die Teilsequenz der Formel

| | | | | | | | | | | | | | | |
|---|---|---|---|---|---|---|---|---|---|---|---|---|---|---|
| | | | Leu | Ala | Gln | Met | Arg | Arg | Ile | Ser | Leu | Phe | Ser | 28 |
| Cys | Leu | Lys | Asp | Arg | Arg | Asp | Phe | Gly | Phe | Pro | Gln | Glu | Glu | 42 |
| Phe | Gly | Asn | Gln | Phe | Gln | Lys | Ala | Glu | Thr | Ile | Pro | Val | Leu | 56 |
| His | Glu | Met | Ile | Gln | Gln | Ile | Phe | Asn | Leu | Phe | Ser | Thr | Lys | 70 |
| Asp | Ser | Ser | Ala | Ala | Trp | Asp | Glu | Thr | Leu | Leu | Asp | Lys | Phe | 84 |

11. Verfahren zur Herstellung von IFN-α2(Arg) gemäß Ansprüchen 9 oder 10, dadurch gekennzeichnet, daß ein Mikroorganismus gemäß den Ansprüchen 6 bis 8 kultiviert wird und das so gebildete IFN-α2(Arg) abgetrennt wird.

12. Verfahren zur Herstellung eines Mikroorganismus gemäß den Ansprüchen 6 bis 8, dadurch gekennzeichnet, daß ein Plasmid gemäß Anspruch 3 oder 4 in einen entsprechenden Wirt transformiert wird.

13. Verfahren zur Herstellung einer DNA gemäß Anspruch 2, dadurch gekennzeichnet, daß man die DNA aus einem Plasmid gemäß Anspruch 3 oder 4 isoliert.

**Claims**

1. A polydeoxyribonucleotide coding for IFN-α2(Arg), characterised in that this polydeoxyribonucleotide corresponds to a sequence occurring in the PstI insert of the plasmid designated pBR322(Pst)1F7 which is deposited in E. coli HB101 at the Deutsche Sammlung für Mikroorganismen under DSM Number 2362.

2. A DNA sequence as claimed in claim 1, characterised in that the sequence coding for IFN-α2(Arg) contains the partial sequence of formula

| | | | | | | | | | | | | | | |
|---|---|---|---|---|---|---|---|---|---|---|---|---|---|---|
| | | 120 | | | | | | | | | | | | |
| | | C | CTG | GCA | CAG | ATG | AGG | AGA | ATC | TCT | CTT | TTC | TCC | 153 |
| TGC | TTG | AAG | GAC | AGA | CGT | GAC | TTT | GGA | TTT | CCC | CAG | GAG | GAG | 195 |
| TTT | GGC | AAC | CAG | TTC | CAA | AAG | GCT | GAA | ACC | ATC | CCT | GTC | CTC | 237 |
| CAT | GAG | ATG | ATC | CAG | CAG | ATC | TTC | AAT | CTC | TTC | AGC | ACA | AAG | 279 |
| GAC | TCA | TCT | GCT | GCT | TGG | GAT | GAG | ACC | CTC | CTA | GAC | AAA | TTC | 321 |
| TA | | | | | | | | | | | | | | 323 |

3. A pBR322 plasmid containing in the PstI insert a DNA sequence according to claim 1 or 2.

4. The plasmid of claim 3 designated pBR322(Pst)1F7 deposited in E. coli HB101 at the Deutsche Sammlung für Mikroorganismen under DSM Number 2362.

5. Process for preparing a clone containing a plasmid as claimed in claim 3, characterised in that a corresponding clone bank is prepared from cDNA which had been synthesised with RNA from Namalwa cells induced with Sendai virus and from this clone bank

a) using the tridecanucleotide of formula

5'–dCCTTCTGGAACTG–3'

by hybridisation, a clone which contains this tridecanucleotide and codes for LeIFN-B, C, D, F or G is obtained and

b) subsequently a clone coding for IFN-α2(Arg) is selected by hybridisation with the cDNA insert of a clone obtained according to a).

6. A microorganism containing in a plasmid a polydeoxyribonucleotide coding for IFN–α2(Arg) as claimed in claim 1 or 2.

7. A microorganism as claimes in claim 6 transformed with a plasmid as claimed in claim 3 or 4.

8. E. coli transformed with a plasmid as claimed in claim 3 or 4.

9. The α-interferon designated IFN-α2(Arg) which is coded by a polydeoxyribonucleotide as claimed in claim 1 or 2 and is substantially free from other cellular material.

10. IFN-α2(Arg) as claimed in claim 9, containing, in the region of amino acids 18–8, the partial sequence of formula

| | | | | | | | | | | | | | |
|---|---|---|---|---|---|---|---|---|---|---|---|---|---|
| | | | Leu | Ala | Gln | Met | Arg | Arg | Ile | Ser | Leu | Phe | Ser | 28 |
| Cys | Leu | Lys | Asp | Arg | Arg | Asp | Phe | Gly | Phe | Pro | Gln | Glu | Glu | 42 |
| Phe | Gly | Asn | Gln | Phe | Gln | Lys | Ala | Glu | Thr | Ile | Pro | Val | Leu | 56 |
| His | Glu | Met | Ile | Gln | Gln | Ile | Phe | Asn | Leu | Phe | Ser | Thr | Lys | 70 |
| Asp | Ser | Ser | Ala | Ala | Trp | Asp | Glu | Thr | Leu | Leu | Asp | Lys | Phe | 84 |

11. Process for preparing IFN-α2(Arg) as claimed in claim 9 or 10, characterised in that a microorganism as claimed in claims 6 to 8 is cultivated and the IFN-α2(Arg) thus formed is separated off.

12. Process for preparing a microorganism as claimed in claims 6 to 8, characterised in that a plasmid as claimed in claim 3 or 4 is transformed into a corresponding host.

13. Process for preparing a DNA as claimed in claim 2, characterised in that the DNA is isolated from a plasmid as claimed in claims 3 or 4.

**Revendications**

1. Polydésoxyribonucléotide codant pour l'IFN-α2(Arg), caractérisé en ce que ce polydésoxyribonucléotide correspond à une séquence présente dans l'insert PstI du plasmide désigné par pBR322(Pst)1F7 lequel, dans E. Coli HB101, est déposé à la collection allemande de microorganismes sous le no DSM 2362.

2. Séquence d'ADN suivant la revendication 1, caractérisée en ce que la séquence codant pour l'IFN-α2(Arg) contient la séquence partielle répondant à la formule

| | | | | | | | | | | | | | |
|---|---|---|---|---|---|---|---|---|---|---|---|---|---|
| | | 120 | | | | | | | | | | | |
| | | C | CTG | GCA | CAG | ATG | AGG | AGA | ATC | TCT | CTT | TTC | TCC | 153 |
| TGC | TTG | AAG | GAC | AGA | CGT | GAC | TTT | GGA | TTT | CCC | CAG | GAG | GAG | 195 |
| TTT | GGC | AAC | CAG | TTC | CAA | AAG | GCT | GAA | ACC | ATC | CCT | GTC | CTC | 237 |
| CAT | GAG | ATG | ATC | CAG | CAG | ATC | TTC | AAT | CTC | TTC | AGC | ACA | AAG | 279 |
| GAC | TCA | TCT | GCT | GCT | TGG | GAT | GAG | ACC | CTC | CTA | GAC | AAA | TTC | 321 |
| TA | | | | | | | | | | | | | | 323 |

3. Plasmide pBR322 contenant, dans l'insert PstI, une séquence d'ADN suivant la revendication 1 ou 2.

4. Plasmide désigné par pBR322(Pst)1F7 suivant la revendication 3, qui est déposé, dans E. Coli HB101, à la collection allemande de microorganismes sous le no DSM 2362.

5. Procédé de préparation d'un clone contenant un plasmide suivant la revendication 3, caractérisé en ce qu'on prépare à partir d'un ADNc qui a été préparé par synthèse avec de l'ARN provenant de cellules de Namalwa induites par le virus Sendai, une banque de clones adéquate, et en ce qu'on détermine à partir de celle-ci

a) à l'aide du tridécanucloétide répondant à la formule

5'–dCCTTCTGGAACTG–3'

par hybridation, un clone qui contient ce tridécanucléotide et code pour le LeIFN-B, C, D, F ou G et

b) on choisit ensuite un clone codant pour l'IFN-α2(Arg) par hybridation avec l'insert d'ADNc d'un clone obtenu conformément à a).

6. Microorganisme contenant dans un plasmide un polydésoxyribonucléotide codant pour l'IFN-α2(Arg) suivant la revendication 1 ou 2.

7. Microorganisme suivant la revendication 6, transformé par un plasmide suivant la revendication 3 ou 4.

8. E. Coli transformé avec un plasmide suivant la revendication 3 ou 4.

9. Interféron-α désigné par IFN-α2(Arg), qui est codé par un polydésoxyribonucléotide suivant la revendication 1 ou 2, et est essentiellement exempt d'autre matière cellulaire.

10. IFN-α2(Arg) suivant la revendication 9, contenant, dans la région des aminoacides no 18–84, la séquence partielle répondant à la formule

| | | | | | | | | | | | | | | |
|---|---|---|---|---|---|---|---|---|---|---|---|---|---|---|
| | | | Leu | Ala | Gln | Met | Arg | Arg | Ile | Ser | Leu | Phe | Ser | 28 |
| Cys | Leu | Lys | Asp | Arg | Arg | Asp | Phe | Gly | Phe | Pro | Gln | Glu | Glu | 42 |
| Phe | Gly | Asn | Gln | Phe | Gln | Lys | Ala | Glu | Thr | Ile | Pro | Val | Leu | 56 |
| His | Glu | Met | Ile | Gln | Gln | Ile | Phe | Asn | Leu | Phe | Ser | Thr | Lys | 70 |
| Asp | Ser | Ser | Ala | Ala | Trp | Asp | Glu | Thr | Leu | Leu | Asp | Lys | Phe | 84 |

11. Procédé de préparation de l'IFN-α2(Arg) suivant les revendications 9 ou 10, caractérisé en ce qu'on cultive un microorganism suivant les revendications 6 à 8 et en ce qu'on sépare l'IFN-α2(Arg) ainsi formé.

12. Procédé de préparation d'un microorganisme suivant les revendications 6 à 8, caractérisé en ce qu'on transforme un plasmide suivant la revendication 3 ou 4 dans un hôte approprié.

13. Procédé de préparation d'un ADN suivant la revendication 2, caractérisé en ce qu'on isole l'ADN d'un plasmide suivant la revendication 3 ou 4.

Figur 1:

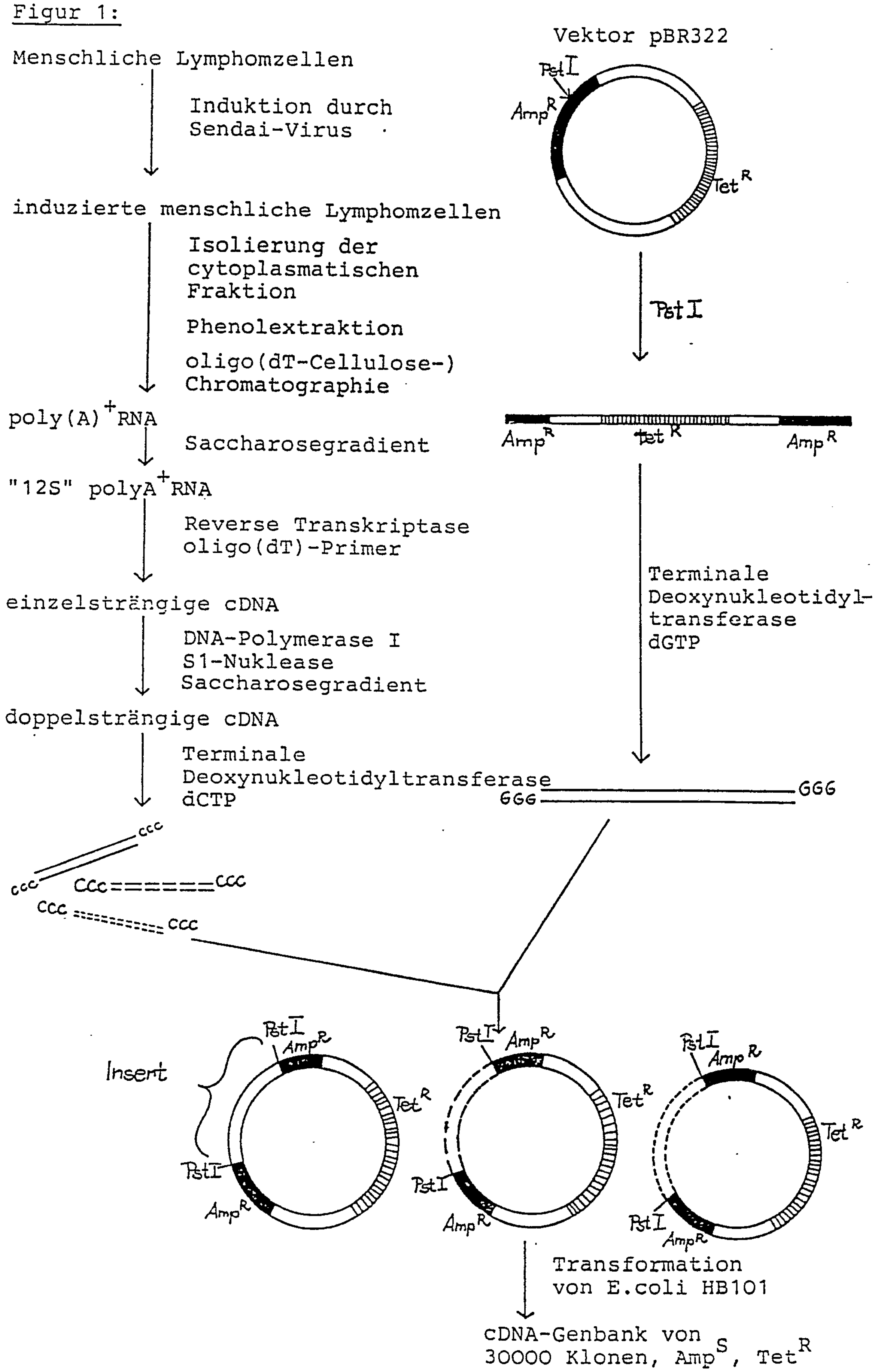
Vektor pBR322
PstI
AmpR
TetR
Menschliche Lymphomzellen
Induktion durch
Sendai-Virus
induzierte menschliche Lymphomzellen
Isolierung der
cytoplasmatischen
Fraktion
Phenolextraktion
oligo(dT-Cellulose-)
Chromatographie
poly(A)+RNA
Saccharosegradient
"12S" polyA+RNA
Reverse Transkriptase
oligo(dT)-Primer
einzelsträngige cDNA
DNA-Polymerase I
S1-Nuklease
Saccharosegradient
doppelsträngige cDNA
Terminale
Deoxynukleotidyltransferase
dCTP
PstI
AmpR
TetR
AmpR
Terminale
Deoxynukleotidyl-
transferase
dGTP
GGG
GGG
ccc
ccc
CCC
CCC
CCC
CCC
Insert
PstI
AmpR
TetR
PstI
AmpR
Transformation
von E.coli HB101
cDNA-Genbank von
30000 Klonen, AmpS, TetR

Figur 2:

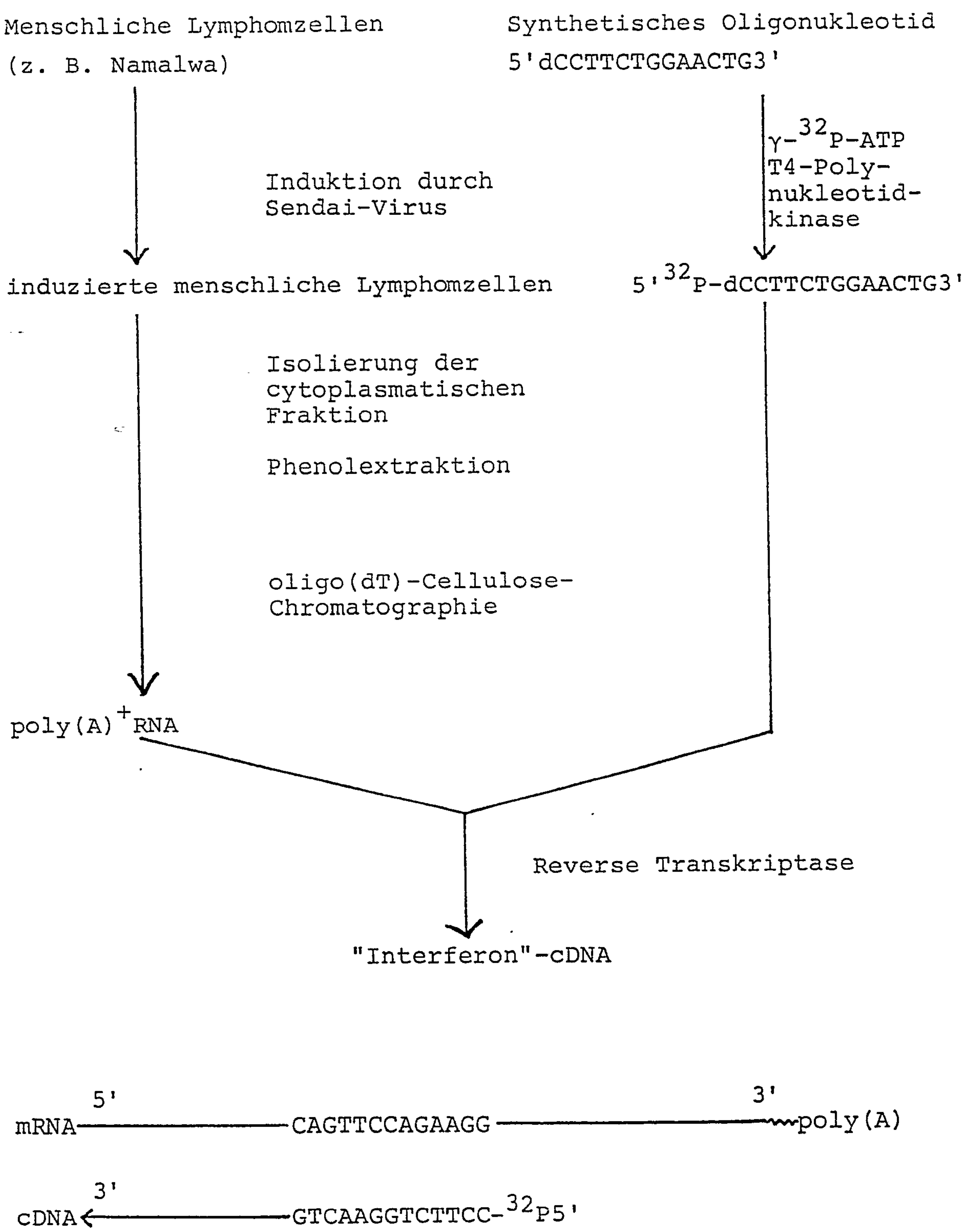
Menschliche Lymphomzellen
(z. B. Namalwa)
Synthetisches Oligonukleotid
5'dCCTTCTGGAACTG3'
Induktion durch Sendai-Virus
$\gamma$-$^{32}$P-ATP
T4-Poly-nukleotid-kinase
induzierte menschliche Lymphomzellen
5'$^{32}$P-dCCTTCTGGAACTG3'
Isolierung der cytoplasmatischen Fraktion
Phenolextraktion
oligo(dT)-Cellulose-Chromatographie
poly(A)$^+$RNA
Reverse Transkriptase
"Interferon"-cDNA
mRNA
5'
CAGTTCCAGAAGG
3'
poly(A)
cDNA
3'
GTCAAGGTCTTCC-$^{32}$P5'

Figur 3:

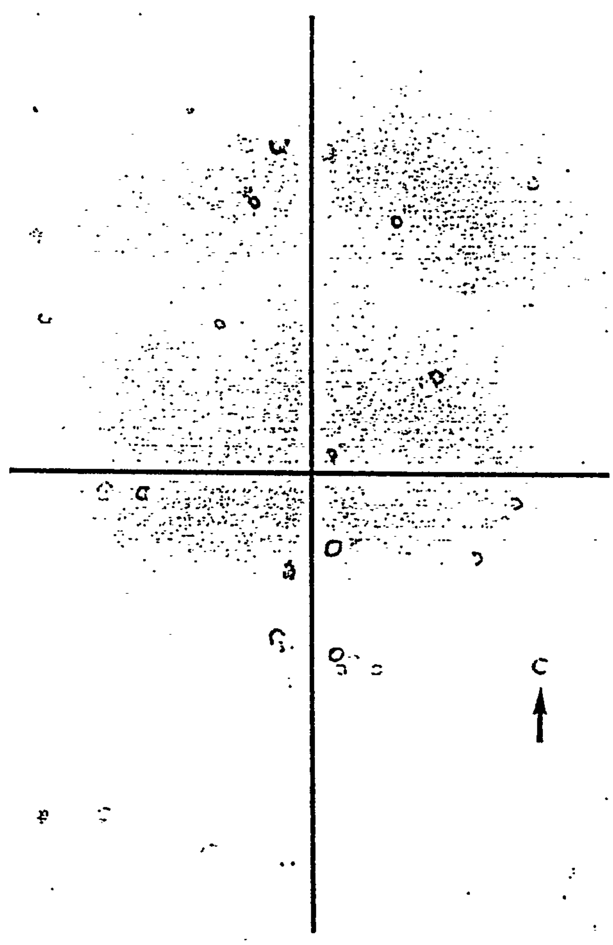

Figur 4:

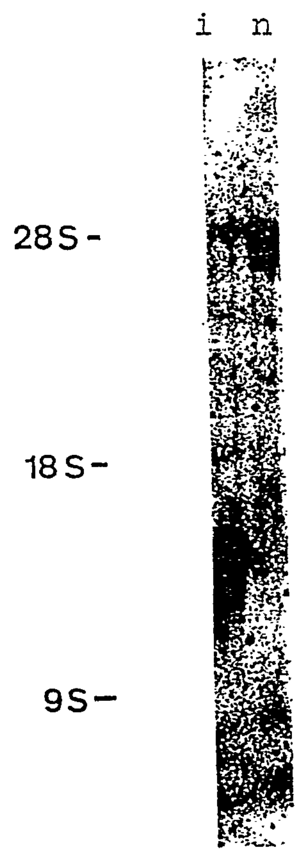

Figur 5:

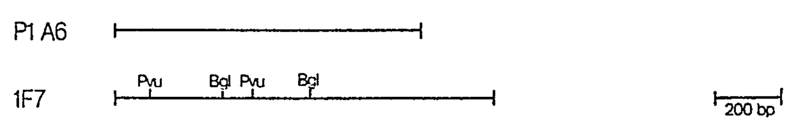

Figur 6:

Klon P1A6:

5'-Ende:......TTATCCATCTCAAGTAGCCTAGCAATATTTGCAACATCCCAATG......
3'-Ende: .....GAGTCGCTTTACATTGTGGTTAATGTAACAATATATGTTCTTC polyA

Klon 1F7:

5'-Ende: ....AGGGTCACCCATTTCAACCAGTCTAGCAGCATCTGCAACATCTACAATG..
3'-Ende: ....TTATATTCAAGATATAAGTAAAAATAAACTTTCTGTAAACC polyA